# EUROPEAN PATENT APPLICATION

(11) **EP 2 078 520 A1**
(43) Date of publication of application: **15.07.2009**
(21) Application number: 07829976.5
(22) Date of filing: 17.10.2007
(51) Int. Cl.: A61H 33/02, A61H 33/14

(54) **CARBON DIOXIDE EXTERNAL APPLICATION DEVICE**

(30) Priority: 18.10.2006 JP 2006283291
(71) Applicant: Neochemir, Inc., Kobe-shi, Hyogo 651-0087 (JP)
(72) Inventor: TANAKA, Masaya, Kobe-shi Hyogo 654-0073 (JP); DAIKYO, Toshiya, Kobe-shi Hyogo 651-0087 (JP)
(74) Representative: Sajda, Wolf E.
(86) International application number: PCT/JP2007/070242
(87) International publication number: WO 2008/047829

(57) **Abstract**

A carbon dioxide external administration device (1) according to the present invention, comprises a sealing enclosure member (2) capable of sealing off a body surface of a human or an animal from the outside air; a supply unit (3) for supplying carbon dioxide to the inside of the sealing enclosure member (2); and a perspiration promoting means (4) for promoting perspiration at the body surface inside the sealing enclosure member (2).

## Description

### TECHNICAL FIELD

The present invention relates to carbon dioxide external administration devices and methods capable of easily obtaining medical effects, cosmetic effects, effects of recovery from fatigue, etc.

### BACKGROUND ART

In recent years, carbon dioxide has been put to use in the fields of medical care, cosmetology, etc. Carbon dioxide is said to have medical effects and cosmetic effects (see Patent Document 1).

Methods for administering carbon dioxide into living bodies include a method for injecting carbon dioxide directly under skin by using an injector. Further, studies have also been conducted on a bath additive in which carbonate and acid are mixed, a circulation type carbonate spring bath system in which carbon dioxide gas (hereinafter, synonymous with "carbon dioxide") tank and a hollow-fiber membrane are used, and so on. Furthermore, Patent Document 2 discloses a poultice for obtaining the effect of stimulating blood circulation by means of carbon dioxide gas. Moreover, Patent Document 1 discloses a carbon dioxide percutaneous and transmucosal absorption composition, which is a viscous composition containing bubble carbon dioxide.

However, in the cases of the above-mentioned bath additive and circulation type carbonate spring bath system, carbon dioxide is dissipated into the air if the temperature of a bathtub is high, and therefore, it is difficult to increase the amount of absorption of carbon dioxide through skin and/or mucous membrane. In order to increase the amount of absorption of carbon dioxide, large amounts of carbonate and acid, or a large amount of carbon dioxide gas is required. In other words, these methods have problems that they are inefficient and uneconomical to achieve effects in attaining medical and cosmetic objectives.

In the case of the above-mentioned poultice, a cloth containing water is superposed on a cloth containing carbonate and organic acid, thereby generating carbon dioxide gas so that the generated carbon dioxide gas is dissolved in the water contained in the cloth, and is utilized as dissolved carbon dioxide gas. However, a reaction between carbonate and organic acid is generally very vigorous, and the amount of carbon dioxide dispersed into the air is larger than the amount of carbon dioxide dissolved in the water; therefore, from the above-mentioned poultice, it is hard to expect medical effects or cosmetic effects resulting from percutaneous and transmucosal absorption of carbon dioxide.

The foregoing carbon dioxide percutaneous and transmucosal absorption composition is capable of obtaining a high concentration of carbon dioxide on skin or on mucous membrane, but a large amount of the foregoing composition must be used for a long period of time in order to obtain satisfactory medical effects or cosmetic effects. Moreover, since the foregoing composition has a high viscosity and has difficulty in being removed from the skin or mucosa after use, the use of the foregoing composition is bothersome.

Therefore, in order to solve the above-described problems, the present inventor has already proposed a carbon dioxide external administration device having a contrivance to prevent dissipation of carbon dioxide, and capable of efficiently, easily and reliably allowing a high concentration of carbon dioxide to be absorbed into a living body (see Patent Document 3).

The carbon dioxide external administration device disclosed in Patent Document 3 is characterized by including: a sealing enclosure member capable of sealing off a body surface from outside air; a supply unit for supplying carbon dioxide to the inside of the sealing enclosure member; and an absorption aid for assisting percutaneous and transmucosal absorption of carbon dioxide inside the sealing enclosure member. Further, this device promotes the absorption of carbon dioxide through the body surface by means of the absorption aid, thereby making it possible to easily obtain carbon dioxide-induced excellent medical effects and cosmetic effects.

Furthermore, Patent Document 4 discloses a carbon dioxide treatment device including: a carbon dioxide supply source; a space-forming member capable of forming a space surrounding a region to be treated; and a passage-forming member for forming a passage through which carbon dioxide is sent from the supply source to the inside of the space.
Patent Document 1: Japanese Unexamined Patent Application Publication JP-A-2000-319 187
Patent Document 2: Japanese Unexamined Patent Application Publication JP-A- 62-286 922
Patent Document 3: International Publication WO2004/002393
Patent Document 4: Japanese Unexamined Patent Application Publication JP-A-2005-058 745

### DISCLOSURE OF THE INVENTION

### PROBLEMS TO BE SOLVED BY THE INVENTION

The former carbon dioxide external administration device described above is capable of efficiently promoting the absorption of carbon dioxide through the body surface by means of the absorption aid. However, according to results of experiments afterward, the following facts were found. Specifically, when percutaneous and transmucosal absorption of carbon dioxide is carried out while perspiration of a person to be treated is promoted, medical effects and cosmetic effects are further improved, and in addition, an effect such as recovery from fatigue can be also obtained.

In view of the above-described facts, an object of the present invention is to provide a carbon dioxide external administration device and a carbon dioxide external administration method, which are capable of further improving medical effects and cosmetic effects, and also capable of obtaining effects of recovery from fatigue, etc.

### SOLUTION TO THE PROBLEMS

As described above, the present inventor has focused attention on a fact that percutaneous and transmucosal absorption of carbon dioxide is very effectively promoted when a body surface (which means a surface of the skin sand mucosa) is perspiring, and thus has reached the completion of the present invention.

Specifically, a carbon dioxide external administration device of the present invention is characterized by including: a sealing enclosure member capable of sealing off a body surface of a human or an animal from outside air; a supply unit for supplying carbon dioxide to an inside of the sealing enclosure member; and perspiration promoting means for promoting perspiration at the body surface inside the sealing enclosure member.

The efficiency of percutaneous and transmucosal absorption of carbon dioxide in gaseous state is generally extremely low. However, as also apparent from examples described later, the following facts were found. Specifically, when a human body is perspiring at a body surface, this perspiring region is sealed off and exposed to carbon dioxide for a certain period of time; then, carbon dioxide dissolved in sweat is efficiently absorbed percutaneously and transmucosally, and medical effects, cosmetic effects, effects of recovery from fatigue, etc. can be obtained in a short period of time.

Further, in the carbon dioxide external administration device of the present invention, sweat itself resulting from perspiration serves as a carbon dioxide-dissolving medium as described above, and therefore, medical effects, cosmetic effects, effects of recovery from fatigue, etc. can be obtained even if no absorption aid is provided in advance in the inside of the sealing enclosure member.

Furthermore, as described above, the carbon dioxide external administration device and method according to the present invention are effective not only to humans but also to animals. However, the following description of the present invention will be centered on humans.

The sealing enclosure member according to the present invention means a member capable of sealing off a body surface that is part of or whole of a human body, and capable of storing a certain amount of gas inside the member. It should be noted that this sealing enclosure member leaks no inside gas or only a small amount of inside gas to the outside.

The carbon dioxide supply unit according to the present invention is not particularly limited as long as the unit can supply gaseous carbon dioxide to the inside of the sealing enclosure member. For example, it is possible to use a unit such as a carbon dioxide gas tank, which is capable of discharging internally-stored carbon dioxide when necessary, or a unit capable of generating and discharging carbon dioxide when necessary by causing a reaction between acid and carbonate.

The perspiration promoting means according to the present invention may be constituted by a pressurizing member for pressurizing gas inside the sealing enclosure member to a pressure exceeding atmospheric pressure. As described in the examples, upon pressurization of the gas inside the sealing enclosure member filled with carbon dioxide to a pressure exceeding atmospheric pressure, a treated person feels a considerable sensation of warmth, and perspires. Accordingly, the pressurizing member in this case functions as the perspiration promoting means for promoting perspiration at the body surface. It should be noted that the perspiration promoting means also includes the case where the body surface, at which perspiration is caused by exercise, bath, etc., is sealed off by the sealing enclosure member.

Methods for pressurizing the gas inside the sealing enclosure member include a method for increasing a supply pressure from the carbon dioxide supply unit. However, this method is uneconomical because the supply amount of carbon dioxide is increased.

Therefore, in the present invention, the pressurizing member is constituted by: a pressurizing enclosure member for surrounding a periphery of the sealing enclosure member; and an air supply unit for supplying air to a space formed between the pressurizing enclosure member and the sealing enclosure member.

In this case, since the pressurizing enclosure member surrounds the periphery of the sealing enclosure member, and air is supplied to the space between both of the enclosure members, the gas inside the sealing enclosure member can be pressurized even if the supply amount of carbon dioxide is not increased. Hence, as compared with the case where pressurization is carried out by increasing the supply amount of carbon dioxide, the gas inside the sealing enclosure member can be more inexpensively pressurized, and treatment by percutaneous and transmucosal absorption of carbon dioxide can be more inexpensively carried out.

Moreover, as the perspiration promoting means according to the present invention, a heating member for heating the inside of the sealing enclosure member to a temperature exceeding the skin temperature, or a humidifying member for increasing the humidity of the inside of the sealing enclosure member to a humidity exceeding the room humidity can be adopted. It should be noted that in the present invention, the inside of the sealing enclosure member means an internal region surrounded by the sealing enclosure member.

As the heating member, a far infrared heater for partially heating a region to be treated, or a hot air heater for heating the entire body of a person to be treated, for example, can be adopted.

The temperature exceeding the skin temperature inside the sealing enclosure member is preferably 25 °C to 45 °C, and is more preferably 30 °C to 43 °C. It should be noted that the skin temperature is preferably 25 °C to 43 °C, and is more preferably 32 °C to 42 °C. It is generally known that tissue cells die at a temperature of 43 °C or more, and therefore, attention must be paid so as not to allow the skin temperature to exceed 43 °C when the present invention is carried out.

As the humidifying member, a spray unit for supplying steam to the inside of the sealing enclosure member can be adopted. Further, the humidifying member also includes the case where a humidifier or the like is connected to the carbon dioxide supply unit to supply humidified carbon dioxide to the inside of the sealing enclosure member, or the case where a humidifier is provided in the inside of the sealing enclosure member. As the humidifier, a hot towel or the like may be used.

The humidity is preferably a relative humidity of 60 % or more, and is more preferably a relative humidity of 75 % or more.

In the present invention, an absorption aid for assisting percutaneous and transmucosal absorption of carbon dioxide inside the sealing enclosure member may further be provided.

The absorption aid includes a carbon dioxide-dissolving medium such as water, alcohols, fats and oils, and waxes, and is applied or affixed, for example, so as to be adhered to the skin or mucosa in accordance with the form, property or the like of the absorption aid, thereby forming, on the skin or mucosa, a layer including the carbon dioxide-dissolving medium. As specific examples of such an absorption aid, various members can be adopted as described in Patent Document 3.

### BRIEF DESCRIPTION OF THE DRAWINGS

- FIG. 1: is a schematic structural diagram of a carbon dioxide external administration device according to a first embodiment.
- FIG. 2: is a schematic structural diagram of a carbon dioxide external administration device according to a second embodiment.
- FIG. 3: is a schematic structural diagram of a carbon dioxide external administration device according to a third embodiment.
- FIG. 4: is a schematic structural diagram of a carbon dioxide external administration device according to a fourth embodiment.
- FIG. 5: is a schematic structural diagram of a carbon dioxide external administration device according to a fifth embodiment.
- FIG. 6: is a schematic structural diagram of a carbon dioxide external administration device according to a sixth embodiment.

### DESCRIPTION OF REFERENCE CHARACTERS

- 2: = sealing enclosure member
- 3: = supply unit
- 4: = perspiration promoting means
- 6: = pressurizing enclosure member
- 11: = second tank (air supply unit)
- 25: = absorption aid

### BEST MODE FOR CARRYING OUT THE INVENTION

Hereinafter, embodiments of the present invention will be described with reference to the drawings. It should be noted that in respective diagrams, the same reference characters denote the same elements or equivalent elements.

A carbon dioxide external administration device 1 according to the present invention includes at least: a sealing enclosure member 2 capable of sealing off a body surface from outside air; a supply unit 3 for supplying carbon dioxide to the inside of the sealing enclosure member 2; and a perspiration promoting means 4 for promoting perspiration at the body surface inside the sealing enclosure member 2.

It is to be noted that the sealing enclosure member 2 does not have to completely seal off the body surface from outside air, but it may be only necessary to continually supply a small amount of carbon dioxide to the sealing enclosure member 2, thereby maintaining the concentration of carbon dioxide inside the sealing enclosure member 2 at or above a determined level.

### FIRST EMBODIMENT

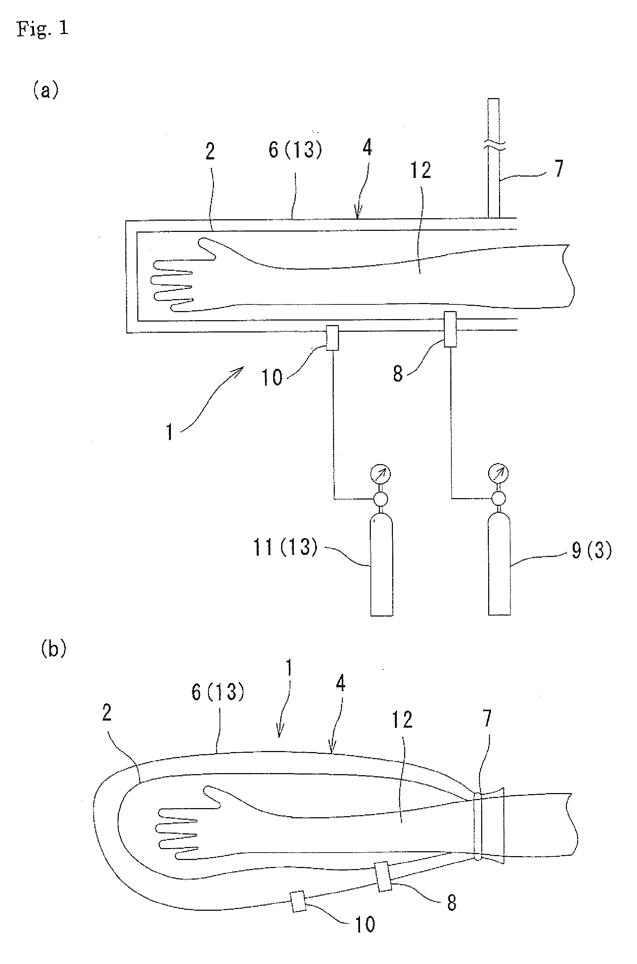

FIG. 1 shows the carbon dioxide external administration device 1 according to a first embodiment of the present invention.

As shown in FIG. 1(a), in the administration device 1 of the present embodiment, the sealing enclosure member 2 is a bag body opened at its one end side, and made of flexible synthetic resin. The sealing enclosure member 2 can cover a body part from a fingertip of a hand to an arm. This bag body is surrounded at its periphery by a pressurizing enclosure member 6, thereby providing a dual inside and outside structure. The pressurizing enclosure member 6 is also a bag body opened at its one end side, and made of synthetic resin. The sealing enclosure member 2 is provided at its opening with a tightening band 7 that is bound around an arm 12, thereby securing the airtightness of the inside of both the enclosure members 2 and 6.

A first coupler 8 having a check valve function is attached to the sealing enclosure member 2. A discharge port of the first coupler 8 airtightly passes through the sealing enclosure member 2 so as to be communicated with the inside of the sealing enclosure member 2. A connection port of the first coupler 8 is protruded to the outside of the pressurizing enclosure member 6. Hence, a hose of a first tank 9 for storing carbon dioxide is connected to the connection port of the first coupler 8, and carbon dioxide is discharged from the first tank 9, thereby enabling the supply of carbon dioxide to the inside of the sealing enclosure member 2. Accordingly, in the present embodiment, the first tank 9 constitutes the carbon dioxide supply unit 3.

A second coupler 10 having a check valve function is attached to the pressurizing enclosure member 6. A discharge port of the second coupler 10 airtightly passes through the pressurizing enclosure member 6 so as to be communicated with the inside of the pressurizing enclosure member 6. A connection port of the second coupler 10 is protruded to the outside of the pressurizing enclosure member 6. Hence, a hose of a second tank 11 for storing air is connected to the connection port of the second coupler 10, and air is discharged from the second tank 11, thereby enabling the supply of air to an space formed between the pressurizing enclosure member 6 and the sealing enclosure member 2. Accordingly, in the present embodiment, the second tank 11 constitutes an air supply unit.

When the administration device 1 of the present embodiment is used, as shown in FIG. 1(a), the arm 12 of a treated person is inserted from his or her fingertip through the opening of the sealing enclosure member 2, and the tightening band 7 is bound around the arm 12, thereby airtightly closing the openings of the sealing enclosure member 2 and the pressurizing enclosure member 6.

In this state, carbon dioxide is supplied from the first tank 9 to the inside of the sealing enclosure member 2, and at the same time, air is supplied from the second tank 11 to the space formed between the sealing enclosure member 2 and the pressurizing enclosure member 6.

As a result, as shown in FIG. 1(b), the inside of the sealing enclosure member 2 is almost filled with carbon dioxide, and the sealing enclosure member 2 is pressurized from outside by the air pressure of the inside of the pressurizing enclosure member 6, thus pressurizing gas inside the sealing enclosure member 2 by a pressure exceeding atmospheric pressure. Therefore, in the present embodiment, the pressurizing enclosure member 6 and the second tank 11 for supplying air to the inside of the pressurizing enclosure member 6 constitute a pressurizing member 13 for pressurizing the gas inside the sealing enclosure member 2 to a pressure exceeding atmospheric pressure.

As apparent from examples described later, if the pressure of the carbon dioxide-containing gas, which is brought into contact with the body surface, is pressurized to a pressure exceeding atmospheric pressure as described above, a sensation of warmth of the entire arm 12 serving as a region to be treated is considerably intensified, and a sensation of warmth is also caused in a region other than the region to be treated, thereby causing perspiration at the arm 12 and the other region of the treated person. Accordingly, the pressurizing member 13 of the present embodiment functions as the perspiration promoting means 4 for promoting perspiration at the body surface inside the sealing enclosure member 2.

It should be noted that the pressure of carbon dioxide inside the sealing enclosure member 2 for causing perspiration at the body surface of the treated person is approximately at a pressure of about 1.1 to about 5 atmospheres. This is because in the case of a pressure of less than 1.1 atmospheres, no pressurizing effect can be obtained, and in the case of a pressure of above 5 atmospheres, further medical effects or cosmetic effects cannon be obtained.

Carbon dioxide dissolves in the sweat of the body surface, caused by the perspiration of the treated person himself or herself, and this dissolved carbon dioxide is effectively absorbed percutaneously and transmucosally. Hence, since the sweat consequently serves as an absorption aid for carbon dioxide, strong medical effects, cosmetic effects, and fatigue recovery effects can be obtained in a short period of time.

Accordingly, the administration device 1 of the present embodiment is capable of obtaining medical effects, cosmetic effects, effects of recovery from fatigue, etc. in a short period of time regardless of whether or not an absorption aid 25 described later is provided in advance inside the sealing enclosure member 2.

Further, in the present embodiment, the pressurizing enclosure member 6 surrounds the periphery of the sealing enclosure member 2, and air is supplied to the space between both of the enclosure members 2 and 6; therefore, the gas inside the sealing enclosure member 2 can be pressurized even if the supply amount of carbon dioxide is not increased. Hence, as compared with the case where pressurization is carried out by increasing the supply amount of carbon dioxide, the inside of the sealing enclosure member 2 can be more inexpensively pressurized, and treatment by percutaneous and transmucosal absorption of carbon dioxide can be more inexpensively carried out.

### SECOND EMBODIMENT

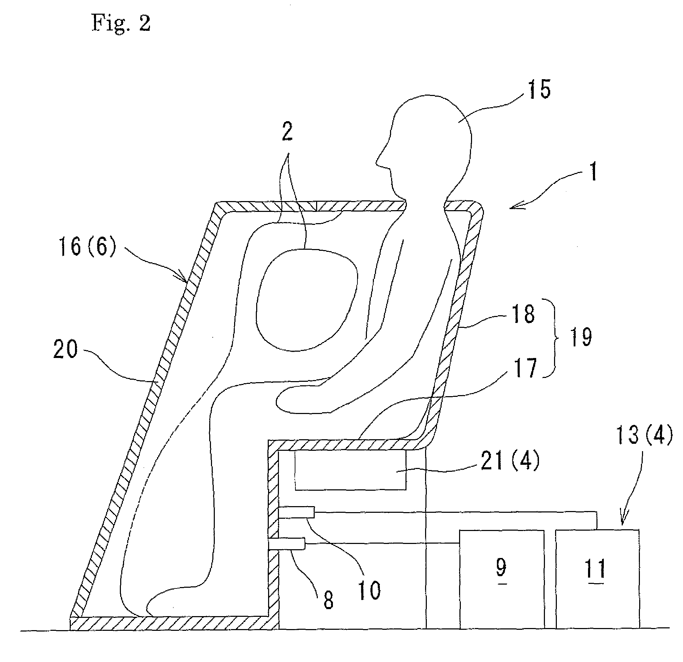

FIG. 2 shows a carbon dioxide external administration device 1 according to a second embodiment of the present invention.

As shown in FIG. 2, in the administration device 1 of the present embodiment, the pressurizing enclosure member 6 is a hard and hollow box body 16, and is capable of accommodating the body of a treated person 15 entirely below his or her neck. The box body 16 includes: a main body part 19 having a seat part 17 and a backrest part 18; and a front lid part 20 for sealing off a front opening of the main body part 19 in an openable and closable manner.

The box body 16 is provided at its inner face side with the sealing enclosure member 2. The sealing enclosure member 2 is a bag body made of flexible synthetic resin, and is capable of accommodating the body of the treated person 15 entirely below his or her neck.

The first coupler 8 and the second coupler 10, each having a check valve function, are attached to a lower part of the box body 16. Further, from the first tank 9 connected to the first coupler 8, carbon dioxide can be supplied to the inside of the sealing enclosure member 2. Furthermore, from the second tank 11 connected to the second coupler 10, air can be supplied to a space formed between the sealing enclosure member 2 and the pressurizing enclosure member 6.

In the present embodiment, at a lower face of the seat part 17 of the box body 16, a heating member 21 including a heating wire heater or the like is provided. The heating member 21 can heat the inside of the sealing enclosure member 2 to a temperature exceeding the skin temperature. It should be noted that the skin temperature of the treated person is at a low temperature (about 25 °C) which is generally lower than a body temperature (about 36 °C), and therefore, it is only necessary for the heating member 21 to be capable of heating the inside of the sealing enclosure member 2 to a temperature exceeding the above-mentioned low temperature.

When the administration device 1 of the present embodiment is used, as shown in FIG. 2, the treated person 15 is allowed to sit on the seat part 17 of the box body 16 with only his or her neck exposed to the outside, and the front lid part 20 is closed to airtightly close the box body 16.

In this state, carbon dioxide is supplied from the first tank 9 to the inside of the sealing enclosure member 2, and at the same time, air is supplied from the second tank 11 to the space formed between the sealing enclosure member 2 and the pressurizing enclosure member 6.

As a result, the inside of the sealing enclosure member 2 is almost filled with carbon dioxide, and the sealing enclosure member 2 is pressurized from outside by the air pressure of the inside of the pressurizing enclosure member 6, thus pressurizing the gas inside the sealing enclosure member 2 by a pressure exceeding atmospheric pressure. Therefore, also in the present embodiment, the pressurizing enclosure member 6 and the second tank 11 for supplying air to the inside of the pressurizing enclosure member 6 constitute the pressurizing member 13 for pressurizing the gas inside the sealing enclosure member 2 to a pressure exceeding atmospheric pressure.

As apparent from the examples described later, if the pressure of the carbon dioxide-containing gas, which is brought into contact with the body surface, is pressurized to a pressure exceeding atmospheric pressure as described above, a sensation of warmth of the entire body below the neck, serving as a region to be treated, is considerably intensified, thereby causing perspiration of the treated person 15, Accordingly, the pressurizing member 13 of the present embodiment also functions as the perspiration promoting means 4 for promoting perspiration at the body surface inside the sealing enclosure member 2.

Furthermore, in the second embodiment, the inside of sealing enclosure member 2 is heated to a temperature exceeding the skin temperature by the heating member 21 provided at the seat part 17, thus facilitating the perspiration of the treated person 15. Therefore, in the present embodiment, the heating member 21 also functions as the perspiration promoting means 4 for promoting perspiration at the body surface.

Carbon dioxide dissolves in the sweat of the body surface, caused by the perspiration of the treated person 15 himself or herself, and this dissolved carbon dioxide is effectively absorbed percutaneously and transmucosally. That is, since the sweat consequently serves as an absorption aid for carbon dioxide, strong medical effects, cosmetic effects, and fatigue recovery effects can be obtained in a short period of time.

As described above, the administration device 1 of the present embodiment is also capable of obtaining medical effects, cosmetic effects, effects of recovery from fatigue, etc. in a short period of time regardless of whether or not the absorption aid 25 described later is provided in advance inside the sealing enclosure member 2.

Further, in the present embodiment, the pressurizing enclosure member 6 surrounds the periphery of the sealing enclosure member 2, and air is supplied to the space between both of the enclosure members 2 and 6; therefore, the gas inside the sealing enclosure member 2 can be pressurized even if the supply amount of carbon dioxide is not increased. Therefore, as compared with the case where pressurization is carried out by increasing the supply amount of carbon dioxide, the inside of the sealing enclosure member 2 can be more inexpensively pressurized, and treatment by percutaneous and transmucosal absorption of carbon dioxide can be more inexpensively carried out.

### OTHER EMBODIMENTS

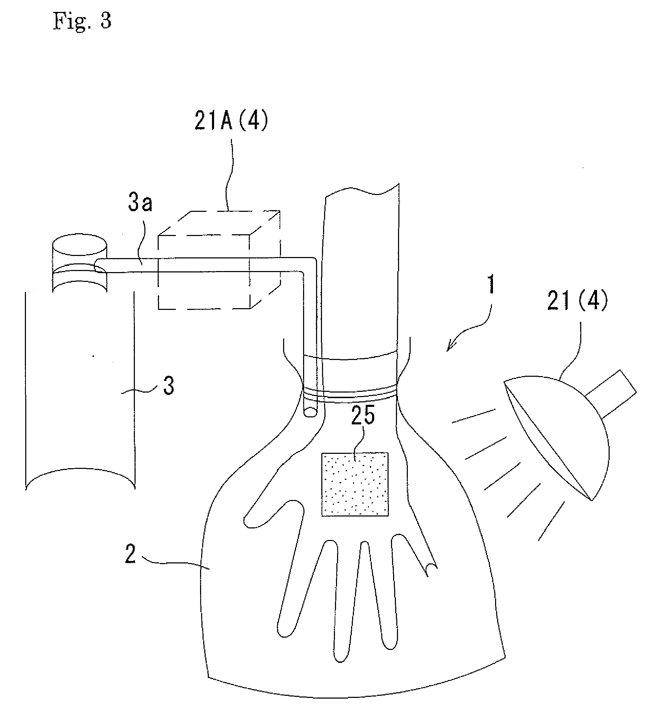

FIGS. 3 to 6 show third to sixth embodiments of the present invention, respectively.

As shown in FIGS. 3 to 6, in the administration device 1 of the present invention, the shape and material of the sealing enclosure member 2 are not particularly limited as long as the member can cover the skin or mucosa and maintain a certain space in which carbon dioxide is held.

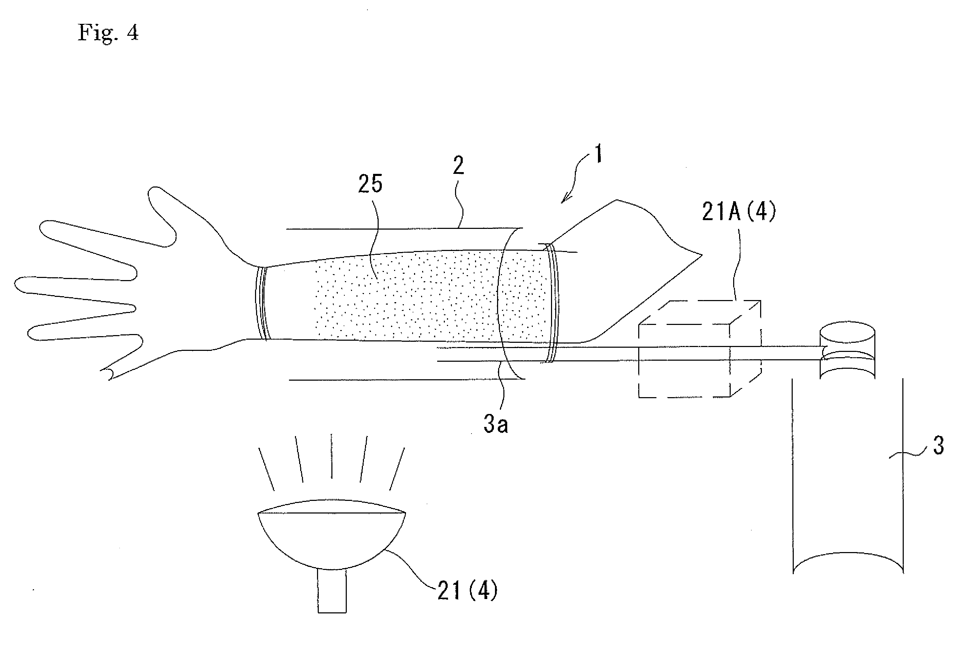

As the shape of the sealing enclosure member 2, it is possible to adopt a bag body capable of covering a hand (or a foot) as shown in FIG. 3, or a tubular bag body that can be wound around an arm (or a foot) as shown in FIG. 4, for example.

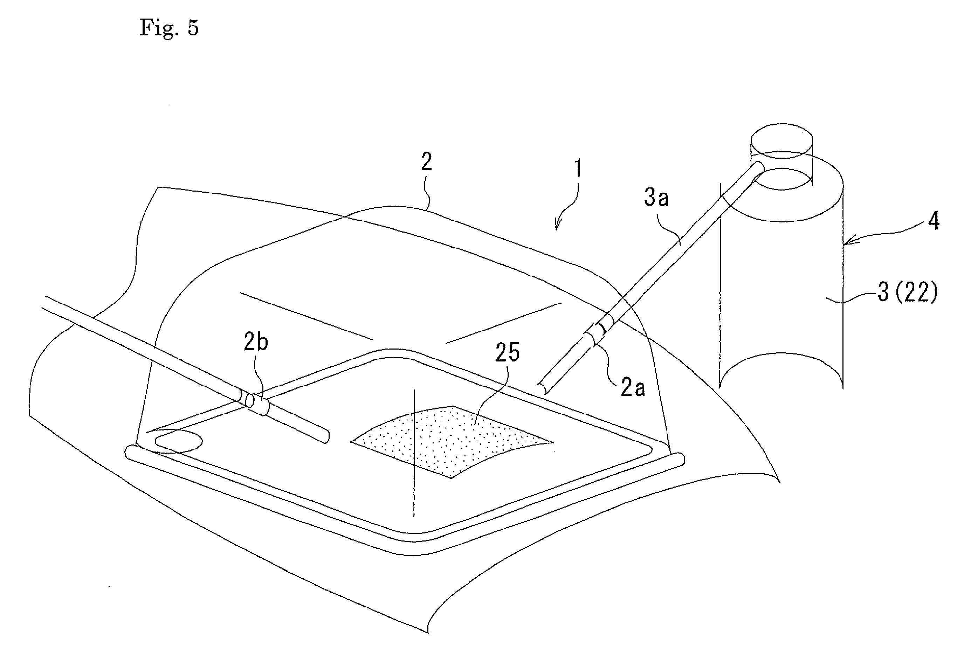

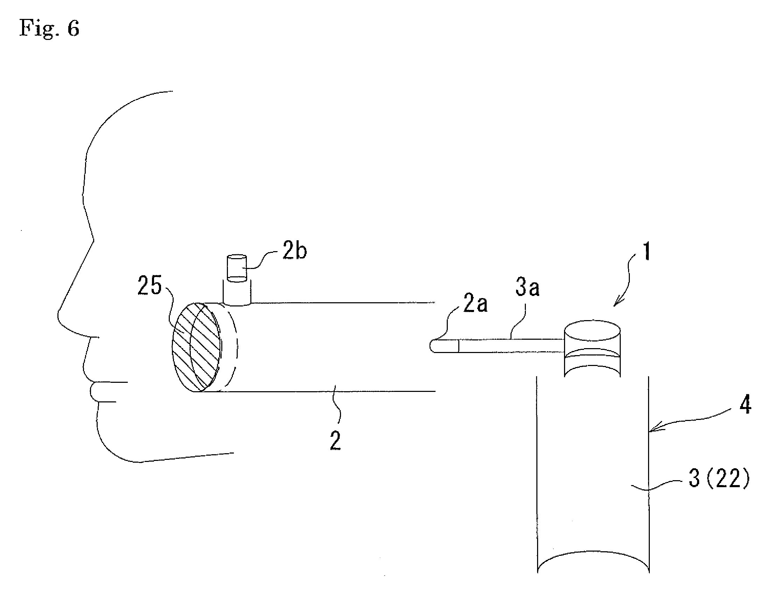

Further, as the shape of the sealing enclosure member 2, it is possible to adopt a container having an opened lower edge that is brought into tight contact with a skin surface with a relatively wide area such as abdomen as shown in FIG. 5, or a cup-shaped cylindrical body, one end of which is opened and the other end of which serves as a connection with the supply unit 3 as shown in FIG. 6. Naturally, the shape of the sealing enclosure member 2 is not limited to these examples.

In the third embodiment of FIG. 3 and the fourth embodiment of FIG. 4, the heating member 21 including a far infrared heater or the like is provided as a constituent element of the administration device 1. The heating member 21 is capable of heating the inside of the sealing enclosure member 2 to a temperature exceeding the skin temperature, and functions as the perspiration promoting means 4 for promoting perspiration of the treated person.

As indicated by virtual lines in FIGS. 3 and 4, a tube 3a, through which carbon dioxide is supplied, may be allowed to pass through a heating member 21 A including a thermoregulated water bath or the like. In this case, when carbon dioxide is supplied from the carbon dioxide supply unit (tank) 3, carbon dioxide heated by the heating member 21 A is supplied to the inside of the sealing enclosure member 2, thereby promoting the perspiration of the treated person. Accordingly, the heating member 21A constitutes the perspiration promoting means 4.

In the fifth embodiment of FIG. 5 and the sixth embodiment of FIG. 6, together with carbon dioxide, steam is enclosed in the inside of the carbon dioxide supply unit (tank) 3 serving as a constituent element of the administration device 1. Furthermore, the supply unit 3 can simultaneously supply carbon dioxide and steam to the inside of the sealing enclosure member 2, and can humidify the inside of the sealing enclosure member 2.

Therefore, in the fifth and sixth embodiments, the carbon dioxide supply unit 3 also functions as a humidifying member 22 for increasing a humidity of the inside of the sealing enclosure member 2 to a humidity exceeding the room humidity. Naturally, in addition to the carbon dioxide supply unit 3, a humidifier (humidifying member) may be connected to the sealing enclosure member 2, thereby separately supplying steam by this humidifier. Upon increase of the humidity of the inside of the sealing enclosure member 2, perspiration through the body surface is promoted, and therefore, the humidifying member 22 functions as the perspiration promoting means 4 for promoting the perspiration of the treated person.

Next, the respective constituent elements of the present invention will be described.

### SEALING ENCLOSURE MEMBER

As described in each of the foregoing embodiments, the constituent material of the sealing enclosure member 2 is not particularly limited as long as it is a gas impermeable material. For example, a material such as metal, plastic, rubber, or glass may be appropriately selected and used in accordance with an objective or an applying region.

If the sealing enclosure member 2 is formed by a nonelastic hard material such as metal or glass (the sealing enclosure member in this case will hereinafter be called a "robust type sealing enclosure member"), the sealing enclosure member 2 preferably has a shape that surrounds an applying region so as to maintain a certain sealed-off space. The skin or mucosa contacting portion preferably has a shape conforming to the applying region so as to prevent the leakage of carbon dioxide. However, an elastic material such as rubber or resin can flexibly conform to the applying region, and is therefore preferably used in combination with the skin or mucosa contacting region. For example, a material such as a viscoelastic gel is more preferably used because it has a higher flexibility and an excellent adherence to the skin or mucosa.

For example, the tube 3a extending from the carbon dioxide supply unit 3 is connected to a gas injection port 2a (see FIGS. 5 and 6) of the robust type sealing enclosure member 2. The gas injection port 2a is not particularly limited as long as it has a structure that prevents leakage of gas, but more preferably includes a check valve for preventing backflow of gas. A gas discharge port 2b (see FIGS. 5 and 6) of the robust type sealing enclosure member 2 may be a gap in the skin or mucosa contacting region, but more preferably includes a check valve for preventing backflow of gas in order to reliably and efficiently carry out the replacement of air inside the sealing enclosure member 2 with carbon dioxide.

If the sealing enclosure member 2 is formed by a material capable of maintaining its form, i.e., a flexible material such as rubber, resin or soft plastic, which is not significantly deformed even if some external force is applied thereto (the sealing enclosure member in this case will hereinafter be called a "flexible type sealing enclosure member"), the skin or mucosa contacting region of the sealing enclosure member 2 is also soft, and can therefore be used as it is. However, since a material such as a viscoelastic gel has a higher flexibility and an excellent adherence to the skin or mucosa, such a material is more preferably used in combination with the skin or mucosa contacting region. It should be noted that also in the flexible type sealing enclosure member 2, the gas injection port and gas discharge port similar to those of the robust type sealing enclosure member 2 can be adopted.

If the sealing enclosure member 2 is formed by a flexible material such as rubber or resin, which has a high elasticity like a balloon (the sealing enclosure member in this case will hereinafter be called an "elastic sealing enclosure member"), the sealing enclosure member 2 can be used in the form of a cylindrical shape or a bag-like shape, for example. In this case, an opening of the cylinder, bag or the like covering a desired region is bound, or an elastic open/close port, to which rubber, spring or the like is attached to its opening, is provided, thus enabling the sealing off of the skin or mucosa.

Further, in the case of the cylindrical elastic sealing enclosure member 2, the applying region of which is an arm, for example, the diameter of the periphery of the cylinder may be set to be substantially similar to or less than that of the periphery of the arm. In this case, the sealing enclosure member 2 may seal off the skin or mucosa in a state where the sealing enclosure member 2 is adhered to the skin or mucosa or the space between the sealing enclosure member 2 and the skin or mucosa is extremely small, and the sealing enclosure member 2 may be expanded by injection of carbon dioxide into the space between the sealing enclosure member 2 and the skin or mucosa, thereby forming a certain space, in which carbon dioxide is held, by the injected carbon dioxide itself.

The gas injection port and gas discharge port of the elastic sealing enclosure member 2 may have the same structures as in the case of the robust type sealing enclosure member 2. However, in the elastic sealing enclosure member 2 in the form of a cylindrical shape, a bag-like shape or the like, the skin or mucosa may be sealed off by the cylinder, bag or the like, inside air may be discharged in advance as much as possible, the tube 3a may be inserted into an opening of the cylinder, bag or the like while air is prevented, to the extent possible, from going inside, and then carbon dioxide may be injected (see FIGS. 3 and 4).

If the sealing enclosure member 2 is formed by a foldable sheet-like or film-like material (the sealing enclosure member in this case will hereinafter be called a "sheet type sealing enclosure member"), the sealing enclosure member 2 can be used in the form of a cylindrical shape, a bag-like shape or the like. In this case, an opening of the cylinder, bag or the like covering a desired region is bound, or an elastic open/close port, to which rubber, spring or the like is attached to its opening, is provided, thus enabling the sealing off of the skin or mucosa.

Further, in the case of the cylindrical sheet type sealing enclosure member 2, the applying region of which is an arm (or a foot), for example, the diameter of the periphery of the cylinder may be set to be greater than that of the periphery of the arm. In this case, the sealing enclosure member 2 may seal off the arm, the sealing enclosure member 2 itself may be folded, inside air may be discharged as much as possible, and then the sealing enclosure member 2 may be used while being adhered to the skin or mucosa.

The gas injection port and gas discharge port of the sheet type sealing enclosure member 2 may have the same structures as in the case of the robust type sealing enclosure member 2. However, in the sheet type sealing enclosure member 2 in the form of a cylindrical shape, a bag-like shape or the like, the skin or mucosa may be sealed off by the cylinder, bag or the like, inside air may be discharged in advance as much as possible, the tube may be inserted into an opening of the cylinder, bag or the like while air is prevented, to the extent possible, from going inside, and then carbon dioxide may be injected.

### CARBON DIOXIDE SUPPLY UNIT

In the administration device 1 of the present invention, the carbon dioxide supply unit 3 is not particularly limited, and a commercially available carbon dioxide gas tank or the like, for example, can be used, or a sealed-off container provided with a tube, for example, can be used as follows. Specifically, carbon dioxide is generated by vaporizing dry ice, which is solid carbon dioxide, inside the sealed-off container, or by a reaction between carbonate and acid inside the sealed-off container.

In the administration device 1 of the present invention, it is only necessary for the tube 3a for connecting the sealing enclosure member 2 with the supply unit 3 to prevent leakage of gas to the outside. As the tube 3a, any material such as rubber, resin, metal or glass may be used with no particular limitation as long as it can be molded into a tube.

### CARBON DIOXIDE

In the administration device 1 of the present invention, carbon dioxide to be used is in gaseous state, and the proportion of carbon dioxide in this gas is preferably 10 % or more, and more preferably 30 % or more. A dose of carbon dioxide is preferably 0.1 mg or more, and more preferably 0.3 mg or more per square centimeter of the skin or mucosa.

### ABSORPTION AID

As shown in each of the embodiments of FIGS. 3 to 6, in the administration device 1 of the present invention, in addition to the adoption of the perspiration promoting means 4, the carbon dioxide absorption aid 25 may additionally be adopted. Examples of a material used for the absorption aid 25 are as follows, and include: a sheet-like material; a viscous material; alcohols; fats and oils; and waxes.

The above-mentioned sheet-like material is not particularly limited as long as the material can be impregnated with a liquid containing at least water and can be affixed to the skin or mucosa. Examples of the above-mentioned sheet-like material include: a woven fabric or a nonwoven fabric consisting of natural fiber, synthetic fiber and/or semi-synthetic fiber; a semi-permeable membrane such as a cellulose membrane; and a hydrogel sheet consisting of naturally-occurring polymers, synthetic polymers and/or semi-synthetic polymers, and one or more of these can be used.

The above-mentioned liquid containing at least water is not particularly limited, and the liquid may be water itself or may be water in which some substance is dissolved or dispersed as long as the effects of the present invention are not impaired. Further, the above-mentioned liquid containing at least water preferably has a pH value of 7 to 2, or the liquid is more preferably acidic water having a pH value of 6.5 to 4. This is because carbon dioxide is efficiently absorbed percutaneously and transmucosally when dissolved in an acidic solvent having a pH value of 4 or more.

The above-mentioned viscous material may be in liquid form or in semi-solid form as long as the material contains water, does not easily flow down when applied to the skin or mucosa, and has a viscosity of 20 cps or more at 20 °C. Examples of a preparation for the above-mentioned viscous material include: liquid; cream; paste; and gel, and one or more of these can be used.

The above-mentioned viscous material preferably has a pH value of 7 to 2, and is more preferably an acidic viscous material having a pH value of 6.5 to 4.

The above-mentioned viscous material is not particularly limited, but is preferably a material having moisture content as high as possible, and capable of replenishing the skin or mucosa with moisture. As a liquid serving as the preparation, a thickener solution, suspension, or swelling liquid, for example, can be suitably used because a necessary viscosity can be obtained with a small amount, or adherence or adhesion to the skin or mucosa is excellent. Methods for producing these liquids are not particularly limited, and public known methods can be adopted. Cream, paste or gel serving as the preparation is not particularly limited, and can be used by producing it by a public known method.

As the above-mentioned thickener used for the viscous material, one or more thickeners selected from a group consisting of: natural polymers; semi-synthetic polymers; synthetic polymers; and inorganic substances can be used.

Examples of the natural polymers include: plant-derived polymers such as gum arabic, carrageenan, galactan, agar, xanthan gum, quince seed gum, guar gum, tragacanth, pectin, manna, locust bean gum, wheat starch, rice starch, corn starch and potato starch; microorganism- derived polymers such as curdlan, xanthan gum, succinoglucan, dextran, hyaluronic acid and pullulan; and protein-type polymers such as albumin, casein, collagen, gelatin and fibroin, and one or more of these can be used.

Among the above-mentioned natural polymers, gum arabic, carrageenan, xanthan gum, tragacanth, hyaluronic acid, pullulan, pectin, mannan, and locust bean gum are preferable in terms of affinity for the skin or mucosa and the like, and furthermore, carrageenan and pectin are more preferable in terms of sense of use and the like.

Examples of the semi-synthetic polymers include: cellulosic polymers such as ethylcellulose, carboxymethyl cellulose, carboxymethylethylcellulose, carboxymethyl starch, croscarmellose, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose and methylhydroxypropylcellulose; starch-type polymers such as pregelatinized starch, partially pregelatinized starch, carboxymethyl starch, dextrin, methyl starch, starch-acrylic acid copolymers and cellulose-acrylonitrile graft copolymers; alginate-type polymers such as sodium alginate and propylene glycol alginate; and other polysaccharide-type polymers such as sodium chondroitin sulfate and sodium hyaluronate, and one or more of these can be used.

Among the above-mentioned semi-synthetic polymers, sodium alginate, propylene glycol alginate, carboxymethylcellulose sodium, dextrin, and sodium hyaluronate are preferable in terms of affinity for the skin or mucosa and the like, and furthermore, sodium alginate, propylene glycol alginate, and sodium hyaluronate are more preferable in terms of sense of use and the like.

Examples of the synthetic polymers include: carboxyvinyl polymer; sodium polyacrylate; polyamine; polyacrylamide; polyvinylacetal diethylaminoacetate; polyvinyl alcohol; polyvinylpyrrolidone; polyethylene glycol; polyethylene oxide; poly(meth)acrylic acid; and polyvinyl methyl ether, and one or more of these can be used.

Among the above-mentioned synthetic polymers, carboxyvinyl polymer, sodium polyacrylate, polyacrylamide, polyvinyl alcohol, and polyvinylpyrrolidone are preferable in terms of affinity for the skin and mucosa and the like, and furthermore, polyvinyl alcohol, polyvinylpyrrolidone, and carboxyvinyl polymer are more preferable in terms of sense of use and the like.

Examples of the inorganic substances include: hydrated silicon dioxide; light anhydrous silicic acid; colloidal alumina; bentonite; and laponite, and one or more of these can be used.

The above-mentioned alcohols are not particularly limited as long as they are in liquid form or in semi-solid form at normal temperature, and are not easily vaporized at human skin temperature. Examples of the above-mentioned alcohols include: monohydric alcohols such as isopropyl alcohol and 1-butanol; and polyhydric alcohols such as ethylene glycol, diethylene glycol, polyethylene glycol, propylene glycol, dipropylene glycol, 1,3-butanediol, 1,2-pentanediol, isoprene glycol, glycerol, diglycerol, triglycerol and tetraglycerol, and one or more of these can be used.

A method for using the alcohols vaporized at high temperature is not particularly limited. The above-mentioned alcohols may be used in accordance with physical properties, etc. thereof by being directly applied or sprayed onto the skin or mucosa, or by being impregnated into a nonwoven fabric or the like so as to be affixed to the skin or mucosa.

The above-mentioned fats and oils are not particularly limited as long as they are in liquid form or in semi-solid form at normal temperature, and can be relatively thinly applied to the skin or mucosa. Examples of the above-mentioned fats and oils include: natural vegetable fats and oils such as avocado oil, avocado butter, olive oil, sesame oil, safflower oil, soybean oil, camellia oil, sunflower oil and macadamia nut oil; and animal fats and oils such as squalane, mink oil, beef fat, lard, chicken fat and horse fat, and one or more of these can be used.

A method for using the above-mentioned fats and oils is not particularly limited. The above-mentioned fats and oils may be used in accordance with physical properties, etc. thereof by being directly applied or sprayed onto the skin or mucosa, or by being impregnated into a nonwoven fabric or the like so as to be affixed to the skin or mucosa.

The above-mentioned waxes are not particularly limited as long as they are in liquid form or in semi-solid form at normal temperature, and can be relatively thinly applied to the skin or mucosa. Examples of the above-mentioned waxes include: jojoba oil; carnauba wax; candelilla wax; beeswax; and lanolin, and one or more of these can be used.

A method for using the above-mentioned waxes is not particularly limited. The above-mentioned waxes may be used in accordance with physical properties, etc. thereof by being directly applied or sprayed onto the skin or mucosa, or by being impregnated into a nonwoven fabric or the like so as to be affixed to the skin or mucosa.

When the absorption aid 25 is applied to the skin or mucosa in the case of the administration device 1 of the present invention, it is preferable that a film of a carbon dioxide-dissolving medium is formed on the skin or mucosa as thin as possible. If the film of the carbon dioxide-dissolving medium is too thick, it takes time for carbon dioxide to be dissolved in the medium, and to be diffused and absorbed into the skin or mucosa, and the absorption efficiency of carbon dioxide might be degraded.

However, if the film of the carbon dioxide-dissolving medium to be formed is too thin when the vaporization temperature of the carbon dioxide-dissolving medium is relatively low, the medium might be vaporized or evaporated and lost from the skin or mucosa due to the skin temperature during absorption of carbon dioxide; therefore, the amount of the carbon dioxide-dissolving medium to be used must be adjusted.

Raw materials commonly used for external preparations or cosmetics can be mixed into the absorption aid 25, and such raw materials can be more suitably used in order to obtain medical effects, cosmetic effects or effects of recovery from fatigue. Examples of such raw materials include: a perfume; a colorant; a surfactant; an oil; a moisturizer; alcohols; a preservative; an antioxidant; a sequestering agent; an anti-coloring agent; an ultraviolet absorbing/scattering agent; vitamins; amino acids; arbutin; kojic acid; a nutrient; an anti-inflammatory agent; a vasodilator; a hormonal agent; an astringent; an antihistamine; a microbicide; a sebum inhibiting agent; a keratin removing/dissolving agent; an anti-seborrheic agent; and an antipruritic.

The administration device 1 of the present invention is applicable not only to humans but also to animals. Animals to which the administration device is applied are not particularly limited as long as they have sweat glands. Specifically, examples of such animals include horses and cattle. For animals having substantially few sweat glands, such as dogs and cats, percutaneous and transmucosal absorption of carbon dioxide can be promoted by using the administration device 1 of the present invention including one or both of: the heating member for heating the inside of the sealing enclosure member to a temperature exceeding the skin temperature; and the humidifying member for increasing the humidity of the inside of the sealing enclosure member to a humidity exceeding the room humidity. The pressurization of gas inside the sealing enclosure member to a pressure exceeding atmospheric pressure is also effective for animals having substantially few sweat glands.

Also for primates, poikilothermal animals and the like, the above-described method for animals having sweat glands or animals having substantially few sweat glands is employed as necessary, and the administration device 1 of the present invention is used, thus making it possible to easily obtain medical effects, cosmetic effects and the like resulting from carbon dioxide.

Hereinafter, the present invention will be more specifically described with examples (test examples) of the present invention presented. Naturally, the present invention will not be limited to these examples.

### EXAMPLE 1

A 51-year-old male test subject took a bath at 42 °C for 15 minutes, and wiped his entire body with a bath towel after taking a bath. The perspiration of the test subject continued slightly, and the entire body was moistly wet. 3 grams of an absorption aid in liquid form was immediately applied to the entire left foot of the test subject in this state, and the left foot was covered with an 80-cm-long polypropylene bag-like sealing enclosure member provided with a check valve.

An opening of the sealing enclosure member was tightly closed at a region at the base of a thigh by a cord attached to the sealing enclosure member, and an end of the cord was bonded to the sealing enclosure member by an adhesive tape, thereby sealing off the left foot of the test subject. Next, a tube was inserted into the check valve, and carbon dioxide was injected therethrough from a high-pressure tank into the inside of the sealing enclosure member, thereby expanding the sealing enclosure member. It should be noted that the injection of carbon dioxide was stopped at the time when the air pressure of the inside of the sealing enclosure member became equivalent to atmospheric pressure.

Since the temperature of carbon dioxide discharged from the high-pressure tank is low, the test subject felt slightly cool at the skin surface of his foot during the injection of carbon dioxide, but felt that not only his entire left foot but also his entire body were warming at the instant of stopping the injection.

In particular, the test subject felt a strong sensation of warmth at the nape of his neck and his back, and felt that his shoulder stiffness and neck stiffness were alleviated. Subsequently, carbon dioxide was injected to expand the sealing enclosure member so that the air pressure of the inside of the sealing enclosure member exceeds atmospheric pressure; then, the test subject felt that the sensation of warmth in his entire left foot was further increased, and also felt that the skin temperature was further significantly increased. At the same time, the test subject felt that his shoulder stiffness and neck stiffness were further alleviated. In addition, the test subject felt that a slight headache he had before this test was alleviated.

A test similar to the above was carried out on a 46-year-old female test subject; then, the similar effects were obtained.

### EXAMPLE 2

3 grams of an absorption aid in liquid form was applied to the entire right arm of a 51-year-old male test subject, and this arm was covered with a polypropylene bag-like sealing enclosure member provided with a check valve.

An opening of the sealing enclosure member was tightly closed by a cord attached to the sealing enclosure member, and an end of the cord was bonded to the sealing enclosure member by an adhesive tape, thereby sealing off the right arm of the test subject. Next, a tube was inserted into the check valve, and carbon dioxide was injected therethrough from a high-pressure tank into the inside of the sealing enclosure member, thereby expanding the sealing enclosure member. It should be noted that the injection of carbon dioxide was stopped at the time when the air pressure of the inside of the sealing enclosure member became equivalent to atmospheric pressure.

Since the temperature of carbon dioxide discharged from the high-pressure tank is low, the test subject felt slightly cool at the skin surface of his right arm during the injection of carbon dioxide, but felt that his entire right arm was warming at the instant of stopping the injection.

Next, a tester pressed the sealing enclosure member with both hands so that the air pressure of the inside of the sealing enclosure member exceeds atmospheric pressure; then, the test subject felt that a sensation of warmth in his entire right arm was significantly increased, and also felt a sensation of warmth in his entire upper body. Again, carbon dioxide was injected into the inside of the sealing enclosure member so that the air pressure of the inside of the sealing enclosure member becomes equivalent to atmospheric pressure; then, the test subject felt that his entire right arm was warming, but did not feel that the sensation of warmth was spreading over his entire upper body.

Next, the tester pressed the sealing enclosure member with both hands similarly to the above; then, ounce again, the test subject felt that the sensation of warmth in his entire right arm was significantly increased, felt a sensation of warmth in his entire upper body, and perspired under his armpit.

A similar test was carried out on a 46-year-old male test subject and a 29-year-old female test subject; then, the similar results were obtained. In particular, a similar test was carried out on a 54-year-old male test subject; then, the test subject remarked that his severe shoulder stiffness was cured and he felt his right arm lighter by the carbon dioxide injection carried out five times.

Further, also in the case where a similar test was carried out with a femoral region set as a test region, the similar results were obtained irrespective of age and sex of test subjects. It should be noted that the wider the application area of the absorption aid, i.e., the wider the area of percutaneous absorption of carbon dioxide, the stronger the sensation of warmth in the entire body.

### EXAMPLE 3

5 grams of an absorption aid in liquid form was applied to the entire both feet of each test subject who is a male jockey, and the both feet were covered with a polypropylene bag-like sealing enclosure member provided with a check valve. Then, an opening of the sealing enclosure member was tightly closed by a cord attached to the sealing enclosure member, and an end of the cord was bonded to the sealing enclosure member by an adhesive tape, thereby sealing off each foot of the test subject. It should be noted that each test subject was in a state in which he perspired slightly all over the body immediately after taking a bath following training. Furthermore, the test subjects were four male jockeys of 19, 21, 23 and 27 years old.

Next, a tube was inserted into the check valve, and carbon dioxide was injected into the inside of the sealing enclosure member so that the air pressure of the inside of the sealing enclosure member exceeds atmospheric pressure; then, all of the four test subjects felt sensations of warmth not only in their entire both feet but also in their entire upper bodies. Furthermore, every time the sealing enclosure member was deflated by percutaneous absorption of carbon dioxide inside the sealing enclosure member, carbon dioxide was repeatedly injected so that the air pressure of the inside of the sealing enclosure member exceeds atmospheric pressure, and the percutaneous absorption of carbon dioxide was carried out for 15 minutes in total. As a result, except for the 27-year-old jockey, the three other jockeys felt fatigue recovery effects in which the fatigue of the entire body was relieved as well as foot muscle pain.

### EXAMPLE 4

A 52-year-old male test subject took a bath at 42 °C for 15 minutes, and wiped his entire body with a bath towel after taking a bath. The perspiration of the test subject continued slightly, and the entire body was moistly wet. The left foot of the test subject in this state was covered with an 80-cm-long polypropylene bag-like sealing enclosure member provided with a check valve without the use of any absorption aid. Then, a test similar to that carried out in EXAMPLE 1 was carried out. The room relative humidity was 58 %, but the relative humidity of the inside of the sealing enclosure member became 80 % after 2 minutes from the start of injection of carbon dioxide, and the entire left foot of the test subject was reddened due to vasodilatation. At this time, the test subject felt a strong sensation of warmth in his left foot. Moreover, the relative humidity of the inside of the sealing enclosure member was 88 % after 5 minutes from the start of injection of carbon dioxide, and the test subject felt that his entire body was further warming. At the same time, the test subject felt that his shoulder stiffness and neck stiffness were alleviated. Subsequently, carbon dioxide was injected into the inside of the sealing enclosure member so that the air pressure of the inside of the sealing enclosure member exceeds atmospheric pressure, thereby expanding the sealing enclosure member; then, the test subject felt that not only the sensation of warmth in his left foot but also the sensation of warmth in his entire body were further increased, and slight perspiration of the test subject was observed at his back and under his armpit. At the same time, the test subject felt that his shoulder stiffness and neck stiffness were further alleviated.

### EXAMPLE 5

A 27-year-old male test subject rode an exercise bike for 10 minutes. The test subject perspired slightly all over the body. Next, the test subject used a training machine "MYORET" to cause fatigue in quadriceps femoris muscles in his both feet. The test subject perspired more heavily. After the end of the training, the left foot of the test subject was covered with an 80-cm-long polypropylene bag-like sealing enclosure member provided with a check valve without the use of any absorption aid. Then, a test similar to that carried out in EXAMPLE 1 was carried out. No treatment was taken on the right foot of the test subject. With the use of MYORET, the degree of fatigue of the quadriceps femoris muscles of the test subject was measured; then, it was found that the recovery from fatigue of the left foot was faster than the recovery from fatigue of the right foot.

### EXAMPLE 6

The left foot of a 52-year-old male test subject was covered with an 80-cm-long polypropylene bag-like sealing enclosure member provided with a check valve without the use of any absorption aid. Then, a test similar to that carried out in EXAMPLE 1 was carried out. The room relative humidity was 60 %, and the relative humidity of the inside of the sealing enclosure member at the start of the test was 65 %. Carbon dioxide was injected into the inside of the sealing enclosure member so that the air pressure of the inside of the sealing enclosure member does not exceed atmospheric pressure; then, the test subject felt no sensation of warmth in his left foot. Furthermore, no reddening of the skin of the test subject was observed.

Next, carbon dioxide was injected into the inside of the sealing enclosure member so that the air pressure of the inside of the sealing enclosure member exceeds atmospheric pressure, thereby expanding the sealing enclosure member; then, the left foot of the test subject perspired, and the relative humidity of the inside of the sealing enclosure member became 75 %. The test subject felt a sensation of warmth in his entire left foot. Furthermore, reddening of the skin of the test subject was observed.

### EXAMPLE 7

A hot towel of 90 °C was put into the inside of an 80-cm-long polypropylene bag-like sealing enclosure member provided with a check valve, and the sealing enclosure member was sealed off for 1 minute. The room relative humidity was 60 %, but the relative humidity of the inside of the sealing enclosure member became 100 %. Next, the left foot of a 52-year-old male test subject was covered with the sealing enclosure member without the use of any absorption aid. Then, a test similar to that carried out in EXAMPLE 1 was carried out. Immediately after the injection of carbon dioxide into the inside of the sealing enclosure member, the test subject felt a sensation of warmth in his left foot, and the skin of the test subject was reddened. Then, after 1 minute, the test subject felt a sensation of warmth in his entire body, and the test subject perspired under his armpit. The test subject felt that fatigue of his left foot and shoulder stiffness were alleviated.

Subsequently, carbon dioxide was injected into the inside of the sealing enclosure member so that the air pressure of the inside of the sealing enclosure member exceeds atmospheric pressure, thereby expanding the sealing enclosure member; then, the test subject felt that not only the sensation of warmth in his left foot but also the sensation of warmth in his entire body were further increased. At the same time, the test subject felt that fatigue of his left foot and shoulder stiffness were further alleviated.

As apparent from EXAMPLES 1 to 7, upon percutaneous and transmucosal absorption of carbon dioxide while perspiration of a treated person (test subject) is promoted, medical effects and cosmetic effects can be further improved, and effects of recovery from fatigue, etc. can be obtained.

### INDUSTRIAL APPLICABILITY

The carbon dioxide external administration devices and methods according to the present invention are industrially very useful because the devices and methods are capable of easily obtaining medical effects, cosmetic effects, effects of recovery from fatigue, etc.

## Claims

1. A carbon dioxide external administration device comprising:
- a sealing enclosure member capable of sealing off a body surface of a human or an animal from the outside air;
- a supply unit for supplying carbon dioxide into the inside of the sealing enclosure member; and
- a perspiration promoting means for promoting perspiration at the body surface inside the sealing enclosure member.

2. The carbon dioxide external administration device according to claim 1, wherein the perspiration promoting means comprises a pressurizing member for pressurizing gas inside the sealing enclosure member to a pressure exceeding atmospheric pressure.

3. The carbon dioxide external administration device according to claim 2, wherein the pressurizing member comprises: a pressurizing enclosure member for surrounding a periphery of the sealing enclosure member; and an air supply unit for supplying air to a space formed between the pressurizing enclosure member and the sealing enclosure member.

4. The carbon dioxide external administration device according to claim 1, wherein the perspiration promoting means comprises a heating member for heating gas inside the sealing enclosure member or the skin or mucosa to a temperature exceeding the skin temperature.

5. The carbon dioxide external administration device according to claim 1, wherein the perspiration promoting means comprises a humidifying member for increasing the humidity of the inside of the sealing enclosure member to a humidity exceeding the room humidity.

6. The carbon dioxide external administration device according to claim 1, wherein the device further comprises, inside the sealing enclosure member, an absorption aid for assisting percutaneous and transmucosal absorption of carbon dioxide.

7. A carbon dioxide external administration method comprising:
sealing off a body surface of a human or an animal from the outside air by a sealing enclosure member; and
supplying, in this state, carbon dioxide into the inside of the sealing enclosure member while perspiration at the body surface is promoted, thereby causing percutaneous and transmucosal absorption of carbon dioxide through the body surface.
